# NEW EUROPEAN PATENT SPECIFICATION

(11) **EP 0 463 101 B2**
(45) Date of publication and mention of the opposition decision: **19.03.2003**
(45) Mention of the grant of the patent: 12.01.1994
(21) Application number: 90905821.6
(22) Date of filing: 21.03.1990
(51) Int. Cl.: C07K 7/06, A61K 38/03, G01N 33/564, G01N 33/68

(54) **VACCINATION AND METHODS AGAINST DISEASES RESULTING FROM PATHOGENIC RESPONSES BY SPECIFIC T CELL POPULATIONS**
IMPFUNG UND METHODEN GEGEN KRANKHEITEN, DIE VON PATHOLOGISCHEN REAKTIONEN DER SPEZIFISCHEN T-ZELLEN ABSTAMMEN
VACCINATION ET PROCEDES CONTRE DES MALADIES RESULTANT DES REPONSES PATHOGENIQUES PAR DES POPULATIONS DE CELLULES T SPECIFIQUES

(30) Priority: 21.03.1989 US 326314; 18.07.1989 US 382085; 18.07.1989 US 382086
(43) Date of publication of application: 02.01.1992
(73) Proprietor: THE IMMUNE RESPONSE CORPORATION, Carlsbad, CA 92008 (US)
(72) Inventor: HOWELL, Mark, D., San Diego, CA 92126 (US); BROSTOFF, Steven, W., Carlsbad, CA 92008 (US); CARLO, Dennis, J., Rancho Santa Fe, CA 92067 (US)
(74) Representative: UEXKÜLL & STOLBERG
(86) International application number: US9001516
(87) International publication number: WO90011294

(56) References cited:
- WO-A-86/06413
- NATURE, vol. 308, March 1984; Y. YANAGI et al., pp. 145-149#
- RESEARCH IMMUNOLOGY, vol. 140, no. 2, 1989; W.E. BIDDISON et al., pp. 212-215#
- IMMUNOL. RESEARCH, vol. 8, no. 2, 1989; C.R. ROSS et al., pp. 81-97#
- CHEMICAL ABSTRACTS, vol. 105, no. 1, 07 July 1986, Columbus, OH (US); S.F. SCHLUTER et al., p. 464, no. 4767q#
- SCIENCE, vol. 246, no. 4930, 1989; M.D. HOWELL et al., pp. 668-670#
- LETTERS TO NATURE, vol. 341, no. 6242, 12 October 1989; A.A. VANDENBARK et al., pp. 541-544#

## Description

### BACKGROUND OF THE INVENTION

This invention relates to the immune system and, more specifically, to methods of modifying pathological immune responses.

Higher organisms are characterized by an immune system which protects them against invasion by potentially deleterious substances or microorganisms. When a substance, termed an antigen, enters the body, and is recognized as foreign, the immune system mounts both an antibody-mediated response and a cell-mediated response. Cells of the immune system termed B lymphocytes, or B cells, produce antibodies which specifically recognize and bind to the foreign substance. Other lymphocytes termed T lymphocytes, or T cells, both effect and regulate the cell-mediated response resulting eventually in the elimination of the antigen.

A variety of T cells are involved in the cell-mediated response. Some induce particular B cell clones to proliferate and produce antibodies specific for the antigen. Others recognize and destroy cells presenting foreign antigens on their surfaces. Certain T cells regulate the response by either stimulating or suppressing other cells.

While the normal immune system is closely regulated, aberrations in immune response are not uncommon. In some instances, the immune system functions inappropriately and reacts to a component of the host as if it were, in fact, foreign. Such a response results in an autoimmune disease, in which the host's immune system attacks the host's own tissue. T cells, as the primary regulators of the immune system, directly or indirectly effect such autoimmune pathologies.

Numerous diseases are believed to result from autoimmune mechanisms. Prominent among these are rheumatoid arthritis, systemic lupus erythematosus, multiple sclerosis, Type I diabetes, myasthenia gravis and pemphigus vulgaris. Autoimmune diseases affect millions of individuals world-wide and the cost of these diseases, in terms of actual treatment and expenditures and lost productivity, is measured in billions of dollars annually. At present, there are no known effective treatments for such autoimmune pathologies. Usually, only the symptoms can be treated, while the disease continues to progress, often resulting in severe debilitation or death.

In other instances, lymphocytes replicate inappropriately and without control. Such replication results in a cancerous condition known as a lymphoma. Where the unregulated lymphocytes are of the T cell type, the tumors are termed T cell lymphomas. As with other malignancies, T cell lymphomas are difficult to treat effectively.

Thus there exists a long-felt need for an effective means of curing or ameliorating T cell mediated pathologies. Such a treatment should ideally control the inappropriate T cell response, rather than merely reducing the symptoms. The present invention satisfies this need and provides related advantages as well.

### Summary of the Invention

The present invention provides vaccines and a means of vaccinating a mammal so as to prevent or control specific T cell mediated pathologies or to treat the unregulated clonal replication of T cells. The vaccine is composed of a T cell receptor (TCR) or a fragment thereof corresponding to a TCR present on the surface of T cells mediating the pathology. The vaccine fragment can be a peptide corresponding to sequences of TCRs characteristic of the T cells mediating said pathology.

Means of determining appropriate amino acid sequences for such vaccines are also provided. The vaccine is administered to the mammal in a manner that induces an immune response directed against the TCR of T cells mediating the pathology, wherein the fragment comprises a V region sequence, or a V(D)J junctional or a J junctional region sequence or mixtures thereof and wherein entire T cells and membrane fractions from entire T cells are excluded. This immune response down regulates or deletes the pathogenic T cells, thus ablating the disease pathogenesis.

The invention additionally provides a specific β-chain variable region of the T cell receptor, designated Vβ17, which is central to the pathogenesis of rheumatoid arthritis (RA). Also provided are means to detect. prevent and treat RA.

### Detailed Description of the Invention

The invention relates to vaccines and their use for preventing or ameliorating T cell-mediated pathologies, such as autoimmune diseases and T cell lymphomas. Vaccination provides a specific and sustained treatment which avoids problems associated with other potential avenues of therapy.

The present invention relates to the use of an immunogenically effective amount of a T cell receptor or fragment thereof corresponding to a T cell receptor present on the surface of T cells mediating a pathology or an unregulated clonal replication, wherein the fragment comprises a V region sequence, or a V(D)J junctional or a J junctional region sequence or mixtures thereof and wherein entire T cells and membrane fractions from entire T cells are excluded, for the preparation of a vaccine for preventing or treating said T cell mediated pathology or an unregulated T cell clonal replication in a mammal.

As used herein, the term "fragment" is intended to cover such fragments in conjunction with or combined with additional sequences or moieties, as for example where the peptide is coupled to other amino acid sequences or to a carrier. The terms "fragment" and "peptide" can, therefore, be used interchangeably since a peptide will be the most common fragment of the T cell receptor. According to the present invention, the fragment comprises a V region sequence, or a V(D)J junctional or a J junctional region sequence or mixtures thereof, wherein entire T cells and membrane fractions from entire T cells are excluded.

Reference herein to a "fragment or portion of the T cell receptor" does not mean that the composition must be derived from intact T cell receptors. Such "fragments or portions" can be produced by various means well-known to those skilled in the art, such as for example manual or automatic peptide synthesis or methods of cloning.

As used herein when referring to the relationship between peptide fragments of the invention and sequences of TCRs, "corresponding to" means that the peptide fragment has an amino acid sequence which is sufficiently homologous to the TCR sequence to stimulate an effective regulatory response in the individual. The sequence need not be identical to the TCR sequence, however, as shown in Examples II and III.

By "immunogenically effective" is meant an amount of the T cell receptor or fragment thereof which, is effective to elicit an immune response to prevent or treat a T cell mediated pathology or an unregulated T cell clonal replication in the individual. Obviously, such amounts will vary between species and individuals depending on many factors. For example, higher doses will generally be required for an effective immune response in a human compared with a mouse.

As used herein, "Vβ17" refers to a specific β-chain variable region of a T cell receptor (TCR). Vβ17 has the amino acid sequence MSNQVLCCVVLCFLGANTVDGGITQSPKYLFRKEGQNVTLSCEQNLNHDAMYWYRQDPGQGLRLIYYSQIVNDFQKGDIAEGYSVSREKKESFPLTVTSAQKNPTAFYLCASS. The hypervariable and junctionai regions are most useful for vaccines. One hypervariable region of Vβ17 especially useful is the CDR2 region which has the amino acid sequence SQIVNDFQK. Modifications in this sequence which do not affect the ability of the receptor to act as an immunogen to stimulate the desired immune response are also included in the definition. The variable region can be joined with any D and J segment of the TCR. Further, immunogenicly representative fragments of Vβ17 are also included in the definition of "Vβ17."

As used herein, "binding partner" means a compound which is reactive with a TCR. Generally. this compound will be a Major Histocompatibility Antigen (MHC) but can be any compound so long as when the TCR is bound in the normal course. T cell activation or proliferation occurs.

As used herein, "ligand" means any molecule that reacts to form a complex with another molecule.

As used herein, "selectively binds" means that a molecule binds to one type of molecule but not substantially to other types of molecules. In relation to Vβ17 "selective binding" indicates binding to Vβ17 containing TCRs but not substantially to other TCRs which lack Vβ17.

The immune system is the primary biological defense of the host (self) against potentially pernicious agents (non-self). These pernicious agents may be pathogens, such as bacteria or viruses, as well as modified self cells, including virus-infected cells, tumor cells or other abnormal cells of the host. Collectively, these targets of the immune system are referred to as antigens. The recognition of antigen by the immune system rapidly mobilizes immune mechanisms to destroy that antigen, thus preserving the sanctity of the host environment.

The principal manifestations of an antigen-specific immune response are humoral immunity (antibody mediated) and cellular immunity (cell mediated). Each of these immunological mechanisms are initiated through the activation of helper (CD4+) T Cells. These CD4+ T cells in turn stimulate B cells, primed for antibody synthesis by antigen binding, to proliferate and secrete antibody. This secreted antibody binds to the antigen and facilitates its destruction by other immune mechanisms. Similarly, CD4+ T cells provide stimulatory signals to cytotoxic (CD8+) T cells which recognize and destroy cellular targets (for example, virus infected cells of the host). Thus, the activation of CD4+ T cells is the proximal event in the stimulation of an immune response. Therefore, elaboration of the mechanisms underlying antigen specific activation of CD4+ T cells is crucial in any attempt to selectively modify immunological function.

T cells owe their antigen specificity to the T cell receptor (TCR) which is expressed on the cell surface. The TCR is a heterodimeric glycoprotein, composed of two polypeptide chains, each with a molecular weight of approximately 45 kD. Two forms of the TCR have been identified. One is-composed of an alpha chain and a beta chain, while the second consists of a gamma chain and a delta chain. Each of these four TCR polypeptide chains is encoded by a distinct genetic locus containing multiple discontinuous gene segments. These include variable (V) region gene segments, junction (J) region gene segments and constant (C) region gene segments. Beta and delta chains contain an additional element termed the diversity (D) gene segment. (Since D segments and elements are found in only some of the TCR genetic loci, and polypeptides, further references herein to D segments and elements will be in parentheses to indicate the inclusion of these regions only in the appropriate TCR chains. Thus, V(D)J refers either to VDJ sequences of chains which have a D region or refers to VJ sequences of chains lacking D regions.)

During lymphocyte maturation, single V, (D) and J gene segments are rearranged to form a functional gene that determines the amino acid sequence of the TCR expressed by that cell. Since the pool of V, (D) and J genes which may be rearranged is multi-membered and since individual members of these pools may be rearranged in virtually any combination, the complete TCR repertoire is highly diverse and capable of specifically recognizing and binding the vast array of binding partners to which an organism may be exposed. However, a particular T cell will have only one TCR molecule and that TCR molecule, to a large degree if not singly, determines the specificity of that T cell for its binding partner.

WO-A-86/06413 discloses on page 51 the peptide M3V corresponding to a fragment of the β chain of the T cell antigen receptor from the T cell lymphoma.

Animal models have contributed significantly to our understanding of the immunological mechanisms of autoimmune disease. One such animal model, experimental allergic encephalomyelitis (EAE), is an autoimmune disease of the central nervous system that can be induced in mice and rats by immunization with myelin basic protein (MBP). The disease is characterized clinically by paralysis and mild wasting and histologically by a perivascular mononuclear cell infiltration of the central nervous system parenchyma. The disease pathogenesis is mediated by T cells with specificity for MBP. Multiple clones of MBP-specific T cells have been isolated from animals suffering from EAE and have been propagated in continuous culture. After in vitro stimulation with MBP, these T cell clones rapidly induce EAE when adoptively transferred to healthy hosts. Importantly, these EAE-inducing T cells are specific, not only for the same antigen (MBP), but also usually for a single epitope on that antigen. These observations indicate that discrete populations of autoaggressive T cells are responsible for the pathogenesis of EAE.

Analysis of the TCRs of EAE-inducing T cells has revealed restricted heterogeneity in the structure of these disease-associated receptors. In one analysis of 33 MBP-reactive T cells, only two alpha chain V region gene segments and a single alpha chain J region gene segment were utilized. Similar restriction of beta chain TCR gene usage was also observed in this T cell population. Only two beta chain V region segments and two J region gene segments were found. More importantly, approximately eighty percent of the T cell clones had identical amino acid sequences across the region of beta chain V-D-J joining. These findings confirm the notion of common TCR structure among T cells with similar antigen specificities and indicate that the TCR is an effective target for immunotherapeutic strategies aimed at eliminating the pathogenesis of EAE.

Various attempts have been made to exploit the antigen specificity of autoaggressive T cells in devising treatment strategies for EAE. For example, passive administration of monoclonal antibodies specific for TCRs present on EAE-inducing T cells has been employed. In the mouse model of EAE, infusion of a monoclonal antibody specific for V_{β}8, the major beta chain V region gene used by MBP-specific T cells, reduced the susceptibility of mice to subsequent EAE induction (Acha-Orbea et al., Cell 54:263-273 (1988) and Urban et al., Cell 54:577-592 (1988)). Similar protection has been demonstrated in rat EAE with monoclonal antibody reactive with an unidentified idiotypic determinant of the TCR on MBP specific T cells (Burns et al., J. Exp. Med. 169:27-39 (1989)). While passive antibody therapy appears to have some ameliorative effect on EAE susceptibility, it is fraught with potential problems. The protection afforded is transient, thus requiring repeated administration of the antibody. Multiple infusions of antibody increases the chances that the host will mount an immune response to the administered antibody, particularly if it is raised in a xenogeneic animal. Further an antibody response to a pathogenic T cell clone represents only one element in the complete immune response and neglects the potential contributions of cellular immunity in resolving the autoreactivity.

The role of cellular immunity in reducing the activity of autoaggressive T cells in EAE has been examined and potential therapies suggested. In a manner similar to the passive antibody approach, regulatory T cells have been derived ex vivo and readministered for immunotherapy. For example, Sun et al., Nature, 332:843-845 (1988), have recently isolated a CD8+ T cell clone from convalescing rats in whom EAE had been induced by adoptive transfer of an MBP-specific CD4+ T cell line. This CD8+ T cell clone displayed cytolytic activity in vitro for the CD4+ T cell used to induce disease. Moreover, adoptive transfer of this CTL clone reduced the susceptibility of recipient rats to subsequent challenge with MBP. Lider et al., Science, 239:181-183 (1988) have also isolated a CD8+ T cell clones with suppressive activity for EAE-inducing T cells. This CD8+ clone was isolated from rats vaccinated with attenuated disease-inducing T cell clones and, though it showed no cytolytic activity in vitro. it could suppress MBP-driven proliferation of EAE-inducing T cells. Although these studies indicate that the CD8+ T cells could downregulate EAE. it is hard to reconcile a major role for these selected CD8+ CTLs in the long-term resistance of the recovered rats since Sedgwick, et al., (Eur. J. Immunol., 18:495-502 (1988)) have clearly shown that depletion of CD8+ cells with monoclonal antibodies does not affect the disease process or recovery.

In the experiments of Sun et al., and Lider et al., described above, the administration of extant derived regulatory T cells overcomes the major obstacle of passive antibody therapy; it permits a regulatory response in vivo of prolonged duration. However, it requires in vitro cultivation with attenuated disease-inducing T cells to develop clones of such regulatory T cells, a costly and labor intensive process. Further, in an outbred population such as humans. MHC non-identity among individuals makes this a highly individualized therapeutic strategy. Regulatory clones need to be derived for each individual patient and then re-administered only to that patient to avoid potential graft versus host reactions.

Direct vaccination with attenuated disease-inducing T cell clones also has been employed as a therapy for EAE. MSP-specific T cells. capable of transferring disease, have been attenuated by gamma irradiation or chemical fixation and used to vaccinate naive rats. In some cases, vaccinated animals exhibited resistance to subsequent attempts at EAE induction (Lider et al., supra: see Cohen and Weiner, Immunol. Today 9:332-335 (1988) for review). The effectiveness of such vaccination, however, is inconsistent and the degree of protection is highly variable. T cells contain a multitude of different antigens which induce an immune response when the whole T cell is administered as a vaccine. This phenomenon has been demonstrated by Offner et al., (J. Neuroimmunol., 21:13-22(1989)), who showed that immunization with whole T cells increased the delayed type hypersensitivity (DTH) response, as measured by ear swelling, to those T cells in an incremental manner as the number of vaccinations increased. However, positive DTH responses were found in both protected and non-protected animals. Rats responded similarly to both the vaccinating encephalitogenic T cells and control T cells. Conversely, vaccination with PPD-specific T cells from a PPD-specific T cell line induced DTH to the vaccinating cells as well as to an encephalitogenic clone even though no protection was observed. The similar response of vaccinated rats to both disease-inducing and control cells, as quantified by delayed-type hypersensitivity (a measure of cell-mediated immunity), indicates that numerous antigens on these T cells are inducing immune responses. Thus, vaccination with attenuated disease-inducing T cells suffers from a lack of specificity for the protective antigen on the surface of that T cell, as well as, variable induction of immunity to that antigen. As a candidate for the treatment of human diseases, vaccination with attenuated T cells is plagued by the same labor intensitveness and need for individualized therapies as noted above for infusion of CD8+ cells.

The present invention provides an effective method of immunotherapy for T cell mediated pathologies, including autoimmune diseases, which avoids many of the problems associated with the previously suggested methods of treatment. By vaccinating, rather than passively administering heterologous antibodies, the host's own immune system is mobilized to suppress the autoaggressive T cells. Thus, the suppression is persistent and may involve any and all immunological mechanisms in effecting that suppression. This multi-faceted response is more effective than the uni-dimensional suppression achieved by passive administration of monoclonal antibodies or extant-derived regulatory T cell clones.

As they relate to autoimmune disease, TCRs or fragments thereof are used corresponding to a TCR present on the surface of T cells that mediate autoimmune diseases are used for the preparation of a vaccine. In this context, whole TCRs substantially purified from T cell clones, individual T cell receptor chains (for example, alpha, beta, etc.) or portions of such chains, either alone or in combination can be used, wherein entire T cells and membrane fractions from entire T cells are excluded. The vaccine prepared by using these TCRs or fragments thereof can be homogeneous, for example, a single peptide, or can be composed of more than one type of peptide, each of which corresponds to a different portion of the TCR. Further, these peptides can be from distinct TCRs wherein both TCRs contribute to the T cell mediated pathology.

In a further specific embodiment, T cell receptors or fragments of the TCR which contain Vβ17 can be used to immunize an individual having rheumatoid arthritis to treat or prevent the disease. The immune response generated in the individual can neutralize or kill T cells having Vβ17 and, thus, prevent or treat the deleterious effects of Vβ17-bearing T cells. Moreover, to the extent that Vβ17 is common to T cell receptors on pathogenic T cells mediating autoimmune diseases in general, such vaccines can also be effective in ameliorating such other autoimmune diseases.

By "substantially pure" it is meant that the TCR is substantially free of other biochemical moieties with which it is normally associated in nature. Alternatively, the vaccines comprise peptides of varying lengths corresponding to the TCR or portions thereof. The peptides can be produced synthetically or recombinantly, by means well known to those skilled in the art. Preferably, the peptide vaccines correspond to regions of the TCR which distinguish that TCR from other nonpathogenic TCRs. Such specific regions can be located within the various region(s) of the respective TCR polypeptide chains, especially a short sequence spanning the V(D)J junction, thus restricting the immune response solely to those T cells bearing this single determinant.

The vaccines are administered to a host exhibiting or at risk of exhibiting an autoimmune response. Definite clinical diagnosis of a particular autoimmune disease warrants the administration of the relevant disease-specific TCR vaccines. Prophylactic applications are warranted in diseases where the autoimmune mechanisms precede the onset of overt clinical disease (for example, Type I Diabetes). Thus, individuals with familial history of disease and predicted to be at risk by reliable prognostic indicators could be treated prophylactically to interdict autoimmune mechanisms prior to their onset.

TCR vaccines can be administered in many possible formulations, in pharmacologically acceptable mediums. In the case of a short peptide, the peptide can be conjugated to a carrier, such as KLH, in order to increase its immunogenicity. The vaccine can be administered in conjunction with an adjuvant, various of which are known to those skilled in the art. After initial immunization with the vaccine, a booster can be provided. The vaccines are administered by conventional methods, in dosages which are sufficient to elicit an immunological response, which can be easily determined by those skilled in the art.

Appropriate peptides to be used for immunization can be determined as follows. Disease-inducing T cell clones reactive with the target antigens are isolated from affected individuals. Such T cells are obtained preferably from the site of active autoaggressive activity such as a lesion in the case of pemphigus vulgaris, central nervous system (CNS) in the case of multiple sclerosis or synovial fluid or tissue in the case of rheumatoid arthritis, or alternatively from blood of affected individuals. The TCR genes from these autoaggressive T cells are then sequenced. Polypeptides corresponding to TCRs or portions thereof that are selectively represented among disease inducing T cells (relative to non-pathogenic T cells) can then be selected as vaccines and made and used as described above.

Alternatively, the vaccines can comprise anti-idiotypic antibodies which are internal images of the peptides described above. Methods of making, selecting and administering such anti-idiotype vaccines are well known in the art. See, for example, Eichmann, et al., CRC Critical Reviews in Immunology 7:193-227 (1987), which is incorporated herein by reference.

### T Cell Pathologies of Malignant Etiology

To illustrate the utility of TCR vaccination, autoimmune disease has been discussed. However, T cell lymphoma is another T cell pathology which would be amenable to this type of treatment. Application of this technology in the treatment of T lymphoma would be conducted in virtually identical fashion. In one respect, however, this technology is more readily applied to T cell proliferative disease since the isolation of the pathogenic T cells is more easily accomplished. Once the clones are isolated, the technology is applied in the manner described herein. Specifically, the TCR genes of the T lymphomas are sequenced, appropriate regions of those TCRs are identified and used as vaccines. The vaccines can comprise single or multiple peptides, and can be administered in pharmacologically acceptable formulations with or without adjuvants by conventional means.

### Multiple Sclerosis

T cells causative of multiple sclerosis (MS) have not previously been identified, though MBP-reactive T cells have been proposed to play a role due to the clinical and histologic similarities between MS and EAE. In rat and mouse models of EAE, MBP-reactive, encephalogenic T cells show striking conservation of β-chain VDJ amino acid sequence, despite known differences in MHC restriction and MBP-peptide antigen specificity. This invention is premised on the observation that a human myelin basic protein (MBP)-reactive T cell line, derived from an MS patient, has a TCR β-chain with a VDJ amino acid sequence homologous with that of β-chains from MBP-reactive T cells mediating pathogenesis in experimental allergic encephalomyelitis (EAE), an animal model of MS. This line is specific for another epitope of MBP. This finding demonstrates the involvement of MBP-reactive T cells in the pathogenesis of MS and demonstrates that TCR peptides similar to those described herein for the prevention of EAE can be appropriate in treating MS.

### Rheumatoid Arthritis

Rheumatoid arthritis (RA) is a T cell mediated autoimmune disease. The invention describes oligoclonal infiltrates of activated Vβ17 T cells in the synovium of rheumatoid arthritis patients. The presence of these T cells in the diseased tissue of all patients examined, their oligoclonality, and the cytotoxic activity of one such T cell for synovial adherent cells, demonstrates a central role for Vβ17 bearing T cells in the pathogenesis of RA.

Activated T cell populations in the synovial tissue of RA patients have been examined by analyzing T cell receptor (TCR) mRNAs isolated from IL-2 receptor positive (IL-2R+) synovial T cells. TCR mRNAs were amplified using a polymerase chain reaction (PCR) protocol designed to amplify human TCR β-chain genes containing virtually any Vβ gene element. In this analysis, oligoclonal Vβ17 rearrangements were found to be enriched in the IL2-R+ population, indicating that Vβ17 T cells are likely involved in the pathogenesis of RA. A CD4+, Vβ17 bearing T cell clone has been isolated from one of the synovial tissue specimens and its in vitro cytotoxicity for synovial adherent cells supports the direct involvement of Vβ17 T cells in RA.

As noted, the invention provides the extremely important discovery that a specific variable region of the β-chain of the TCR, designated Vβ17, is closely associated with rheumatoid arthritis in human subjects. This discovery allows for the detection, prevention and treatment of rheumatoid arthritis using the methodology set out in this invention. Similar therapeutic approaches set out above for EAE can be applied to rheumatoid arthritis by those skilled in the art.

Specifically, the invention provides a method of diagnosing or predicting susceptibility to rheumatoid arthritis in an individual comprising detecting T cells having the β-chain variable region designated Vβ17 in a sample from the individual, the presence abnormal levels of Vβ17-containing T cells indicating rheumatoid arthritis or susceptibility to rheumatoid arthritis. The Vβ17 containing T cell can be qualitatively or quantitatively compared to that of a normal individual. Such diagnosis can be performed by detecting a portion of the Vβ17 which does not occur on non-rheumatoid arthritis associated β-chain variable region T-cell receptors. The Vβ17 can be detected, for example, by contacting the Vβ17 with a detectable ligand capable of specifically binding to Vβ17. Many such detectable ligands are known in the art, e.g. an enzyme linked antibody. Alternatively, nucleotide probes complementary to Vβ17 encoding nucleic acid sequences can be utilized to detect Vβ17 containing T cells, as taught in Example IX.

The invention also provides a method of preventing or treating rheumatoid arthritis comprising preventing the attachment of a Vβ17 containing T-cell receptor to its binding partner. In one embodiment attachment is prevented by binding a ligand to Vβ17. In an alternative embodiment attachment is prevented by binding a ligand to the Vβ17 binding partner. Attachment can be prevented by known methods, e.g. binding an antibody to Vβ17 or the binding portion to physically block attachment.

The following examples are intended to illustrate but not limit the invention.

### EXAMPLE I

### RAT MODEL OF EAE

Female Lewis rats, (Charles River Laboratories, Raleigh-Durham, NC) were immunized in each hind foot pad with 50µg of guinea pig myelin basic protein emulsified in complete Freund's adjuvant The first signs of disease were typically observed 9-11 days post-immunization. Disease severity is scored on a three point scale as follows: I=limp tail; 2=hind leg weakness; 3=hind leg paralysis. Following a disease course of approximately four to six days, most rats spontaneously recovered and were refractory to subsequent EAE induction.

### EXAMPLE II - for information only

### SELECTION AND PREPARATION OF VACCINES

Vaccinations were conducted with a T cell receptor peptide whose sequence was deduced from the DNA sequence of a T cell receptor beta gene predominating among EAE-inducing T cells of BIO.PL/L mice. The DNA sequence was that reported by Urban, et al., supra, which is incorporated herein by reference. A nine amino acid peptide, having the sequence of the VDJ junction of the TCR beta chain of the mouse, was synthesized by methods known to those skilled in the art. The sequence of this peptide is: SGDAGGGYE. (Amino acids are represented by the conventional single letter codes.) The equivalent sequence in the rat has been reported to be: SSD-SSNTE (Burns et al., J. Exp. Med. 169:27-39 (1989)). The peptide was desalted by Sephadex G-25 (Pharmacia Fine Chemicals, Piscataway, NJ) column chromatography in 0.1 M acetic acid and the solvent was subsequently removed by two cycles of lyophilization. A portion of the peptide was conjugated to keyhole limpet hemocyanin (KLH) with glutaraldehyde at a ratio of 7.5 mgs of peptide per mg of KLH. The resulting conjugate was dialyzed against phosphate buffered saline (PBS).

### EXAMPLE III - for information only

### VACCINATION AGAINST EAE

Vaccines used in these studies consisted of free VDJ peptide and also of VDJ peptide conjugated to KLH. These were dissolved in PBS and were emulsified with equal volumes of either (1) incomplete Freund's adjuvant (IFA) or (2) complete Freund's adjuvant (CFA) made by suspending 10 mg/ml heat killed desiccated Mycobacterium tuberculosis H37ra (Difco Laboratories, Detroit, MI) in IFA. Emulsions were administered to 8-12 week old female Lewis rats in a final volume of 100 microliters per animal (50 µl in each of the hind footpads). 5µg of unconjugated VDJ peptide were administered per rat. KLH-VDJ conjugate was administered at a dose equivalent to 10µg of KLH per rat. Twenty-nine days later each rat was challenged with 50 µg of guinea pig myelin basic protein in complete Freund's adjuvant in the front footpads. Animals were monitored daily beginning at day 9 for clinical signs of EAE and were scored as described above. The results are presented in Table I. As can be seen, not only was there a reduced incidence of the disease in the vaccinated individuals, but in those which did contract the disease, the severity of the disease was reduced and/or the onset was delayed. The extent of protection varied with the vaccine formulation, those including CFA as the adjuvant demonstrating the greatest degree of protection.

**TABLE I**

| Animal No. | Vaccination (Adjuvant) | Days After Challenge | | | | | | | | |
|---|---|---|---|---|---|---|---|---|---|---|
| | | 10 | 11 | 12 | 13 | 14 | 15 | 16 | 17 | 18 |
| 1 | VDJ (IFA) | - | - | 2 | 3 | 3 | 3 | - | - | - |
| 2 | " | - | - | 1 | 3 | 3 | 3 | 2 | - | - |
| 3 | " | - | - | - | 3 | 3 | 3 | 2 | - | - |
| | | | | | | | | | | |
| 4 | VDJ (CFA) | - | - | - | - | 1 | 1 | 1 | - | - |
| 5 | " | - | - | - | - | - | - | - | - | - |
| 6 | " | - | - | - | 1 | 3 | 3 | 3 | 2 | - |
| | | | | | | | | | | |
| 7 | KLH-VDJ (CFA) | - | - | - | 1 | 3 | 2 | - | - | - |
| 8 | " | - | - | - | - | 1 | 1 | 1 | 1 | - |
| 9 | " | - | - | - | - | - | - | - | - | - |
| | | | | | | | | | | |
| 10 | KLH-VDJ (IFA) | - | 1 | 3 | 3 | 2 | 2 | 1 | - | - |
| 11 | " | - | - | 3 | 3 | 3 | 3 | 3 | 2 | - |
| 12 | " | - | - | 1 | 3 | 3 | 3 | 3 | - | - |
| | | | | | | | | | | |
| 13 | NONE | 1 | 3 | 3 | 3 | 3 | 1 | - | - | - |
| 14 | " | - | 1 | 3 | 3 | 3 | 1 | - | - | - |
| 15 | " | 1 | 3 | 3 | 3 | 1 | - | - | - | - |
| Scoring: - no signs 1) limp tail 2) hind leg weakness 3) hind leg paralysis | | | | | | | | | | |

### EXAMPLE IV

### Vaccination against EAE with Lewis Rat VDJ peptides

The VDJ peptide used in the previous examples was synthesized according to the sequence of TCR ßchain molecules found on EAE-inducing T cells in B10.PL mice. In addition, peptides were synthesized and tested which correspond to sequences found on encephalitogenic T cells in Lewis rats. These VDJ sequences are homologous with that of B10.PL mice, but not identical. The rat peptides were synthesized according to the DNA sequences reported by Burns, et al. and Chluba, et al., Eur. J. Immunol. 19:279-284(1989). The sequences of these three peptides designated IR1, 2 and 3, are shown below, aligned with the B10.PL mouse sequence used in Examples I through III (VDJ).

VDJ S G D A G G Y E
IR1 C A S S D - S S N T E V F F G K
IR2 C A S S D - S G N T E V F F G K
IR3 C A S S D - S G N - V L Y F G E G S R
IR9b A S S D - S S N T E

The preparation, administration and evaluation of these vaccines were conducted as described in Examples I through III with the following exceptions: 50 µg of the individual VDJ peptides were incorporated into vaccine formulations containing CFA; neither vaccinations in IFA nor vaccinations with peptides conjugated to KLH were conducted. Control animals were untreated prior to MBP challenge as in Example III or were vaccinated with emulsions of PBS and CFA to assess the protective effect of adjuvant alone. The results are shown in Table II below.

**TABLE II**

| Animal No. | Vaccination (Adjuvant) | Days After Challenge | | | | | | | | |
|---|---|---|---|---|---|---|---|---|---|---|
| | | 10 | 11 | 12 | 13 | 14 | 15 | 16 | 17 | 18 |
| 1 | None | - | 1 | 2 | 3 | 3 | 2 | - | - | - |
| 2 | " | 1 | 3 | 3 | 3 | 2 | - | - | - | - |
| 3 | " | - | 2 | 3 | 3 | 3 | 1 | - | - | - |
| | | | | | | | | | | |
| 4 | PBS-CFA | 1 | 2 | 3 | 3 | 3 | - | - | - | - |
| 5 | " | 1 | 2 | 3 | 3 | 3 | - | - | - | - |
| 6 | " | - | 2 | 3 | 3 | 3 | - | - | - | - |
| | | | | | | | | | | |
| 7 | IR1 (50 µg) | - | - | - | 2 | 1 | - | - | - | - |
| 8 | " | - | - | - | - | 1 | 3 | - | - | - |
| 9 | " | - | - | - | 1 | 1 | 1 | 1 | - | - |
| | | | | | | | | | | |
| 10 | IR2 (50µg) | - | - | 1 | 3 | 3 | 3 | - | - | - |
| 11 | " | - | - | - | - | 2 | 2 | 3 | 3 | - |
| 12 | " | - | - | - | - | 1 | - | - | - | - |
| | | | | | | | | | | |
| 13 | IR3 (50µg) | 1 | 3 | 3 | 3 | 2 | - | - | - | - |
| 14 | " | - | - | 2 | 3 | 3 | - | - | - | - |
| 15 | " | - | - | - | - | - | - | - | - | - |
| | | | | | | | | | | |
| 16 | IR9b (50 µg) | - | - | - | - | - | - | - | - | - |
| 17 | " | - | - | - | - | - | - | - | - | - |
| 18 | " | - | - | - | - | - | - | - | - | - |
| 19 | " | | | | | | | | | |
| Scoring: - no signs 1) limp tail 2) hind leg weakness 3) hind leg paralysis | | | | | | | | | | |

As shown in Table II, disease in unvaccinated control animals was observed as early as day 10. Disease was characterized by severe paralysis and wasting, persisted for 4 to 6 days and spontaneously remitted. PBS-CFA vaccinated rats displayed disease courses virtually indistinguishable from those of unvaccinated controls. In contrast, delays in onset were observed in some of the IR1, 2 or 3 vaccinated animals and others showed both delayed onset as well as decreased severity and/or duration of disease. Overall, however, vaccinations with the rat VDJ peptides (IR1-3) were slightly less effective than those with the mouse VDJ peptide (Example III). Vaccination with IR9b, however, afforded complete protection in all four animals in which it was tested. Importantly, no histologic lesions characteristic of disease were found in any of the four animals vaccinated with IR9b indicating that sub-clinical signs of disease were also abrogated.

### EXAMPLE V

### Vaccination with V region specific peptides

A peptide specific for the Vβ8 gene family was tested as a vaccine against EAE. Vβ8 is the most common ßchain gene family used by encephalitogenic T cells in both rats and mice. A peptide was synthesized based on a unique DNA sequence found in the Vβ8 gene, and which is not found among other rat Vβ genes whose sequences were reported by Morris, et al., Immunogenetics 27:174-179 (1988). The sequence of this Vβ8 peptide, designated IR7, is:

IR7 D M G H G L R L I H Y S Y D V N S T E K

The efficacy of this Vβ8 peptide was tested in the Lewis rat model of EAE (Example I) as described in Examples II and III. 50 µg of peptide were tested in CFA. Vaccinations in IFA or with peptide-KLH conjugates were not conducted. The results of these studies are shown in Table III.

**TABLE III**

| Animal No. | Vaccination (Adjuvant) | Days After Challenge | | | | | | | | |
|---|---|---|---|---|---|---|---|---|---|---|
| | | 10 | 11 | 12 | 13 | 14 | 15 | 16 | 17 | 18 |
| 1 | IR7 (50 µg) | - | - | 1 | 2 | 3 | 3 | 3 | - | - |
| 2 | " | - | - | - | - | 1 | 1 | - | - | - |
| 3 | " | - | - | - | - | - | - | - | - | - |
| Scoring: - no signs 1) limp tail 2) hind leg weakness 3) hind leg paralysis | | | | | | | | | | |

The results of vaccinations conducted with the rat Vβ8 peptide are similar to those observed with the mouse and rat IRI, 2 and 3 peptides. Delayed onset as well as decreased severity and duration of disease was observed in one animal. One animal was completely protected.

### EXAMPLE VI

### Vaccination with J region peptides

A peptide was synthesized which corresponds to the J α gene segment, TA39, found among both rat and mouse encephalitogenic T cell receptors. The sequence of this peptide, designated IR5, is:

IR5 R F G A G T R L T V K

The efficacy of the JαTA39 peptide was tested in the Lewis rat model of EAE (Example I) as described in Examples II and III. 50 µg of peptide were tested in CFA. Vaccinations in IFA or with peptide-KLH conjugates were not conducted. The results of these studies are shown in Table IV.

**TABLE IV**

| Animal No. | Vaccination (Adjuvant) | Days After Challenge | | | | | | | | | | |
|---|---|---|---|---|---|---|---|---|---|---|---|---|
| | | 10 | 11 | 12 | 13 | 14 | 15 | 16 | 17 | 18 | 19 | 20 |
| 1 | IR5 (50 µg) | - | - | - | - | - | 2 | 1 | 1 | 1 | 1 | - |
| 2 | " | - | - | - | - | - | - | - | - | - | - | - |
| 3 | " | - | - | - | - | - | - | - | - | - | - | - |
| Scoring: - no signs 1) limp tail 2) hind leg weakness 3) hind leg paralysis | | | | | | | | | | | | |

The results of vaccinations conducted with the rat J α TA39 peptide are more effective than those observed with the mouse VDJ peptide or the Vβ8 peptide. Two of three animals were totally protected and, in the third, disease onset was markedly delayed. Severity was also reduced in this animal though disease persisted for a normal course of 5 days. Importantly, the two animals which were completely protected showed no histologic evidence of T cell infiltration of the CNS. This result indicates that vaccinating with the J_{α}TA39 very efficiently induces a regulatory response directed at encephalitogenic T cells. Even sub-clinical signs of disease were abrogated.

### EXAMPLE VII

### Vaccination with mixtures of TCR peptides

Vaccinations were conducted with a mixture of TCR peptides. This mixture contained 50 µg of each of the peptides IR1, 2, 3 and 5 (the three rat VDJ peptides and the rat JαTA39 peptide).

The efficacy of this peptide mixture was tested in the Lewis rat model (Example I) as described in Examples II and III. Peptides were tested in CFA. Vaccinations in IFA or with peptide-KLH conjugates were not conducted. The results of these studies are shown in Table V.

**TABLE V**

| Animal | Vaccination | Days After Challenge | | | | | | | | |
|---|---|---|---|---|---|---|---|---|---|---|
| No. | (Adjuvant) | 10 | 11 | 12 | 13 | 14 | 15 | 16 | 17 | 18 |
| 4 | IR1, 2, 3, 5 | - | - | - | - | - | - | - | - | - |
| 5 | (50 µg each) | - | - | - | - | - | - | - | - | - |
| 6 | " | - | - | - | - | - | - | - | - | - |
| Scoring: - no signs 1) limp tail 2) hind leg weakness 3) hind leg paralysis | | | | | | | | | | |

The results of vaccinations conducted with the rat JαTA39 and three VDJ peptides are almost as effective as those described for IR9b in Table II. All three animals were totally protected. In addition to the absence of any clinical signs of EAE, two of these three animals were completely free of histological evidence of T cell infiltration into the CNS while the third showed only two small foci of lymphocytic infiltration at the base of the spinal cord.

### EXAMPLE VIII - for information only

### Multiple Sclerosis Vaccine

### Human MBP-reactive T cells

MBP-reactive T cell lines were established from peripheral blood mononuclear cells (PBMC) of nine chronic progressive MS patients and two healthy controls. Cells were maintained in culture by regular stimulation with purified human MBP and irradiated-autologous PBMC for three days followed by four days in IL-2 containing medium.

### PCR Amplification of TCR β-chain genes from MBP-reactive T cell lines

T cells were harvested from log phase cultures and RNAwas prepared, amplified with the Vβ16mer primer and nested Cβ primers for 55 cycles as described in Example IX.

### TCR β-chain sequences of human MBP-reactive T cells

Vβ16mer amplified TCR β-chain genes from human MBP-reactive T cell lines were sequenced using the Cpseq primer. Amplification products were gel purified, base denatured and sequenced from the Cβseq primer. Readable DNA sequence was obtained from 5 of these lines, indicating that predominant T cell clones had been selected by long term in vitro passage. One of these sequences, from the Re cell line (Table VI), possessed a β-chain VDJ amino acid sequence that shared five of the first six and six of nine total residues with the β-chain VDJ amino acid sequence conserved among MBP reactive, encephalogenic T cells in the B10.PL mouse model of EAE. This sequence was not present among the predominant TCR rearrangements found in the remaining four human MBP reactive T cell lines.

To determine if similar sequences were present in the β-chain repertoire of the MBP-reactive T cell lines from other MS patients, PCR amplification was conducted with a degenerate (n=1024) 21-nucleotide primer (VβRe) corresponding to seven amino acids of this sequence (Table VI). RNAs were reversed transcribed and amplified in 20 cycle stage I reactions with the Vβ16mer and Cβext primers. One µl aliquots of these stage I reactions were reamplified for 35 cycles with the VβRe and Cβ int primers. One µl aliquots of these reactions were analyzed by Southern blot hybridization with a ³²P-labeled human Cβ probe. This analysis revealed the 300 bp amplified product in the Re cell line and in one of the other MS patient lines, but not in MBP-reactive T cells from control subjects or in non-MBP reactive human T cell lines and clones. The presence of this sequence in two of the nine MS patient lines tested is compelling. Since this sequence is known to be conserved among encephalogenic T cells in EAE, its detection among MBP-reactive T cells from MS patients demonstrates a role for T cells bearing this determinant in the pathogenesis of MS.

Immunogenic peptides having the sequence SGDQGGNE can be synthesized as shown in Example II and used to immunize human subjects by methods demonstrated in Example III. Such immunizations can result in an effective immune response.

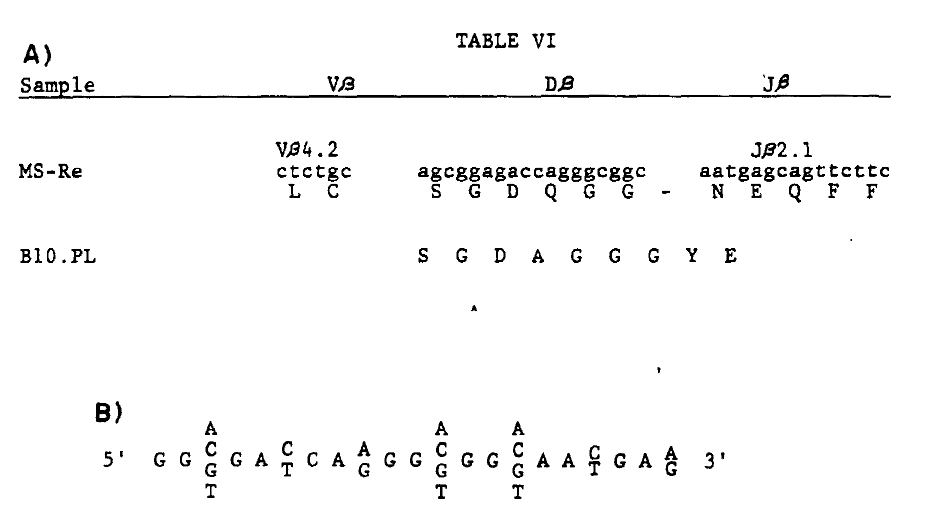

### EXAMPLE IX

### Isolation of Oligoclonal Infiltrates of 11. Activated Vβ17 T Cells in the Synovium of Rheumatoid Arthritis Patients

### T cell preparations from synovial tissue

Synovial tissue specimens were obtained from radiographically proven rheumatoid arthritis patients undergoing joint replacement therapy. Activated T cells were selected using magnetic beads and antibodies reactive with the human IL2-R (αIL2-R) as follows. Synovial tissue was digested for 4 hrs at 37°C in RPMI + 10% Fetal Bovine Serum (FBS) containing 4 mg/ml collagenase (Worthington Biochemical, Freehold, NJ) and 0.15 mg/ml DNAse (Sigma, St. Louis, MO.). Digests were passed through an 80-mesh screen and single cells were collected by Ficoll density gradient centrifugation. Cells at the interface were washed and were incubated at 10⁶/ml for 30 min at 0°C with 5 µg/ml control mouse IgG (Coulter Immunology, Hialeah, FL) in PBS containing 2% FBS (PBS-FBS). Cells were washed three times and incubated for 30 min at 0°C with magnetic beads conjugated to goat anti-mouse IgG (Advanced Magnetics, Cambridge, MA). Beads were magnetically separated and washed three times with PBS-FBS. This preselection with mouse IgG (mIgG) and magnetic beads was used to control for non-specific adsorption of T cells. The cells remaining in the initial suspension were further incubated 30 minutes at 0°C with 5 µg/ml monoclonal mouse IgG reactive with the human T cell IL2-R (Coulter Immunology, Hialeah, FL). Cells were washed and selected with magnetic beads as above. Beads from the IgG preadsorption and the IL2-R antibody selection were immediately resuspended in acidified-guanidiniumphenol-chloroform and RNA prepared as described in Chonezynski and Sacchi, Anal. Biochem. 162:156 (1987), which is incorporated herein by reference. Since RNAS were prepared without in vitro culture of the cells and the accompanying bias that may be induced, they are expected to accurately reflect T cell distributions in synovial tissue at the time of surgical removal. Only half of the mlgG and αIL2-R beads from patient 1012 were immediately processed for RNA. The remainder were cultured for 5 days in RPMI 1640, 5% FBS, 20% HL-1 (Ventrex Laboratories Inc., Portland, ME), 25mM HEPES, glutamine, antibiotics and 20% LAK supernatant (Allegretta et al., Science, 247:718 (1990)), which is incorporated by reference herein, as a source of IL-2. RNA was extracted from cultures of the αIL2-R beads (1012IL2.d5), but not from the 1012mlgG sample as no viable cells were present at the end of the 5 day culture.

A T cell clone was derived from the Ficoll pellet of patient 1008. The cells in the pellet were cultured at 2 x 10⁶/ml in media without IL-2 for two weeks. Non-adherent cells from this culture were cloned by limiting dilution onto autologous synovial cell monolayers. A CD4+ T cell clone 1008.8 was obtained and adapted to culture by regular stimulation with autologous synovial monolayers for 3 days in media without IL-2 followed by a 4 day culture in medium with LAK supernatant.

### Lysis of Synovial Adherent Cells by 1008.8

Lysis of synovial adherent cells by 1008.8 was demonstrated as follows. Synovial cell monolayers were labeled as described in Stedman and Campbell, J. Immunol. Meth. 119:291 (1989), which is incorporated herein by reference, with ³⁵S for use as targets in CTL assays. Cells were typsinized, washed and plated at 2000 cells per well of a 96-well round bottom microtiter plate. 1008.8 cells, cultured for 3 days prior to the assay with synovial adherent cells and medium containing LAK supernatant, were added to the targets at the indicated effector:target ratios. Cultures were incubated overnight at 37°C, centrifuged at 300xg for 2 minutes and radioactivity in 50 µl of the supernatant quantified. Per cent specific lysis was calculated relative to detergent-lysed targets by standard formulas. This clone is cytotoxic for synovial adherent cell targets in CTL assays (Table VII).

**TABLE VII**

| Effector:Target Ratio | % Specific Lysis |
|---|---|
| 5:1 | 7 |
| 10.1 | 16 |
| 25.1 | 32 |

### PCR Amplification of TCR β-chain genes

TCR β-chain genes were amplified with several combinations of the primers shown in Table VIII. The vβ16mer primer is a degenerate Vβ primer (n=256) which is predicted to bind 85% of human TCR β-chain genes at all 16 residues and 95% at 15 residues. This primer has been used to amplify TCR β-chains from more than 25 different human T cell clones, lines or primary tissue preparations. A spectrum of Vβ genes has been sequenced from these amplified DNAs, arguing against a significant bias of the primer for certain Vβ families. Thus, PCR amplification with the Vβ16mer primer facilitates analysis of T cell populations for which a priori knowledge of Vβ gene usage is unavailable.

T cell receptor β-chain genes were amplified in two-stage amplification reactions with nested pairs of the primers shown in Table VIII. RNAs were reverse transcribed for 1 hour at 42°C with 40pmol of the Cpext primer in a 12 µl reaction using conditions described by Hart et al., The Lancet, p. 596 (1988), included by reference herein. Reactions were diluted with a master mix containing 40 pmols of the Vβ16mer primer, nucleotides and reaction buffer as above but without MgCl₂ to give a final Mg⁺² concentration of 3.6 mM. Samples were denatured for 15 minutes at 95°C, 1 unit of heat stable recombinant DNA polymerase (Cetus Corporation, Emeryville, CA, Ampli-taq™) was added and 20 cycles of PCR conducted. Each cycle consisted of a 1 min denaturation at 95°C, a two minute annealing step and a two minute extension at 72°C. The first two cycles were annealed at 37°C and 45°C, respectively, and the remainder at 50°C. One microliter aliquots of these stage I reactions were added to 100 µl stage II amplification reactions (Cetus, Gene-Amp Kit™) containing 100 pmols of the Cβint primer and 100 pmols of the Vβ8, Vβ17 or 5'Cβ primers or 700 pmols of the Vβ16mer primer. Stage II amplifications were conducted as above with a 50°C annealing temperature and without the 37°C and 45°C ramping.

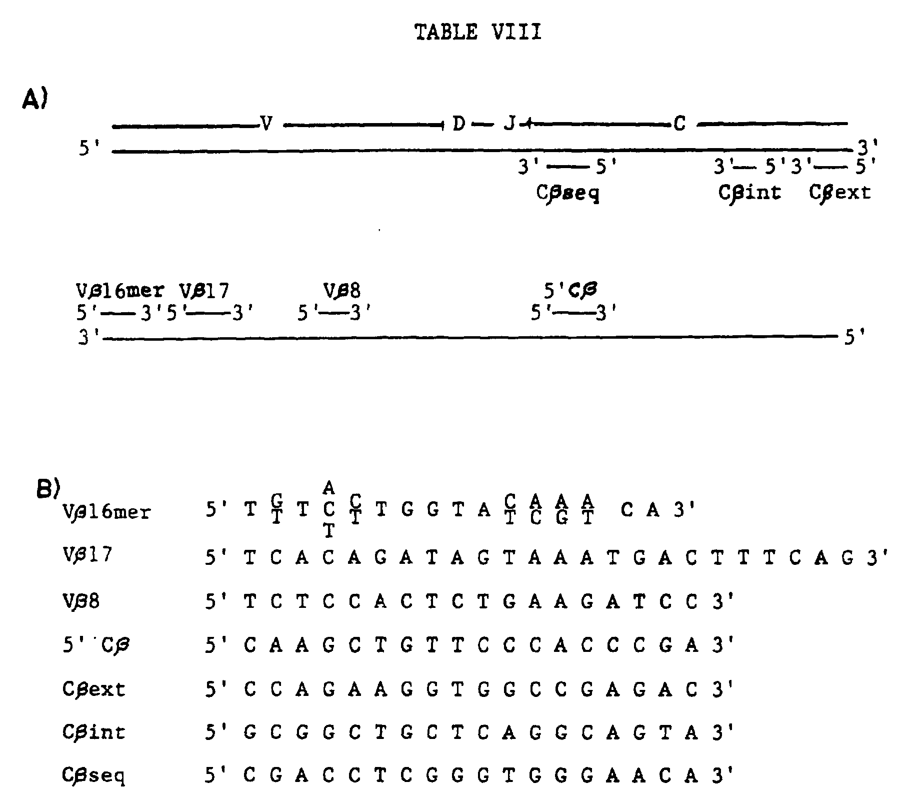

RNA samples from 1012IL2.d5 and 1008.8 cultures were amplified with the Vβ16mer and Cβext primers in stage I reactions and with the Vβ16mer and the Cβint primer in 35 cycle stage II reactions. Reaction products, purified from low melting agarose gel slices with Gene Clean glass beads (Biolol, San Diego, CA), were base denatured and sequenced from the CBseq primer with T7 polymerase (Sequenase, (United States Biochem, Cleveland, OH). A predominate Vβ sequence, corresponding to a single Vβ17 rearrangement Table IX, was clearly readable in the 1012IL2.d5 sample. Other, less frequent rearrangements were detected as faint, uninterpretable background bands in the sequencing gels. Culture of these 1012.IL2 beads in IL2-containing medium without added accessory cells or antigen is not expected to induce de novo activation of T cells. Thus, the predominance of a single Vβ17 rearrangement in this sample reflects in vivo clonal expansion of Vβ17+ T cells in this patient. DNA sequence determination of TCR β-chain DNA amplified from the cytotoxic T cell clone, 1008.8, also revealed a Vβ17 rearrangement (Table IX). The presence of Vβ17 rearrangements in these two different types of synovial T cell samples, derived from two separate RA patients, implicates Vβ17 bearing T cells in the pathogenesis of RA.

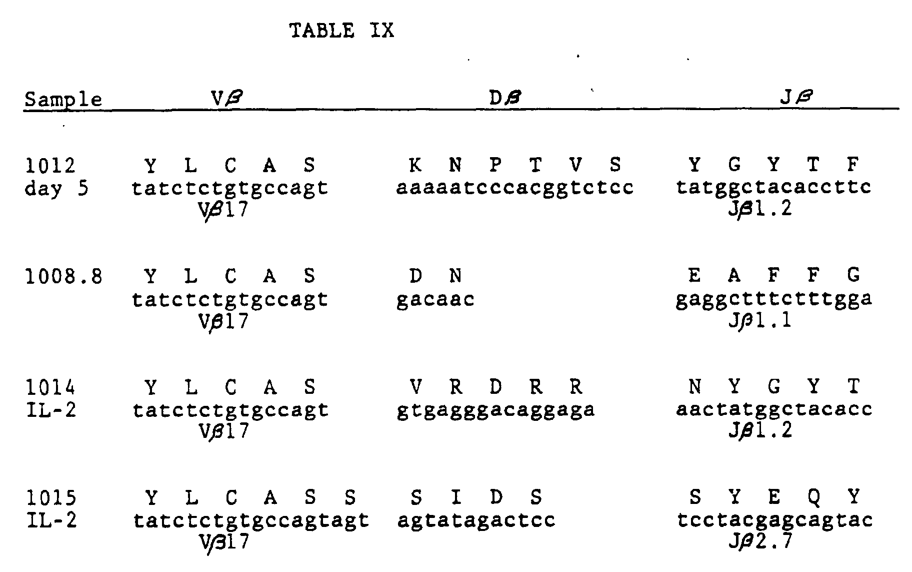

The presence of Vβ17 rearrangements in the remaining synovial RNA samples was assessed by PCR amplification with the Vβ17 specific primer (Table VIII). Vβ17 TCR DNA was amplified from magnetic bead samples derived from each of the seven RA patients. Ethidium bromide staining of electrophoresed reaction products revealed greater Vβ17 amplification in four of the αIL2-R samples than in the corresponding mIgG controls. This enrichment was not a product of the isolation procedure, since amplification of Vβ8 TCRs failed to show differences between MIgG and IL2-R samples.

Vβ17 rearrangements from two of the αIL2-R RNA preparations were amplified with the Vβ17 and Cβint primer pair and the reaction products sequenced with the Cβseq primer. Samples 1014 and 1015 contained single sequences (Table IX), which, like the 1012IL2.d5 sample, demonstrates clonal expansion of Vβ17 T cells in vivo. In contrast, direct sequencing of the rearrangements amplified with the Vβ8 specific primer was not possible due to significant heterogeneity in the β-chain product.

Vβ17 has the amino acid sequence MSNQVLCCVVLCFLGANTVDGGITQSPKYLFRKEGQNVTLS-CEQNLNHDAMYWYRQDPGQGLRLIYYSQIVNDFQKGIAEGYSVSREKKESFPLTVTSAQKNPTAFYLCASS.

### HLA-DR Analysis in Rheumatoid Arthritis Patients

HLA-DR analysis in rheumatoid arthritis patients was performed as follows. DNA from each patient was prepared by boiling 10⁵ synovial cells in 200 µl dH₂O. Ten µl were amplified for 35 cycles in a 100 µl reaction (Cetus, Gene Amp Kit™) containing 100 pmols of each of the DRβ PCR primers (Table X). One-tenth µl of this reaction was reamplified in 10 µls containing only the DRβ2 primer and 17 pmol of α32P-dCTP as the sole source of dCTP for 10 cycles. Reactions were spiked with 200 uM dCTP and chased for 2 cycles. The resulting negative strand probes were hybridized to slot blots containing 10 pmol of the HLA-DR allele specific oligos (positive strands) using conditions previously described by Amor et al., J. Immunol. 138:1947 (1987), which is incorporated herein by reference. The slots were washed twice for 20 minutes with tetramethylammoniumchloride (Wood et al., Proc. Natl. Acad. Sci. USA 82:1585 (1985)) which is incorporated herein by reference) at 65-68°C and exposed to X-ray film.

Each of the patients in this study possessed at least one allele of the HLA-DR genes, DR4w4, DR1, DR4w14 or DR4w15, that are known to predispose for RA (Table X).

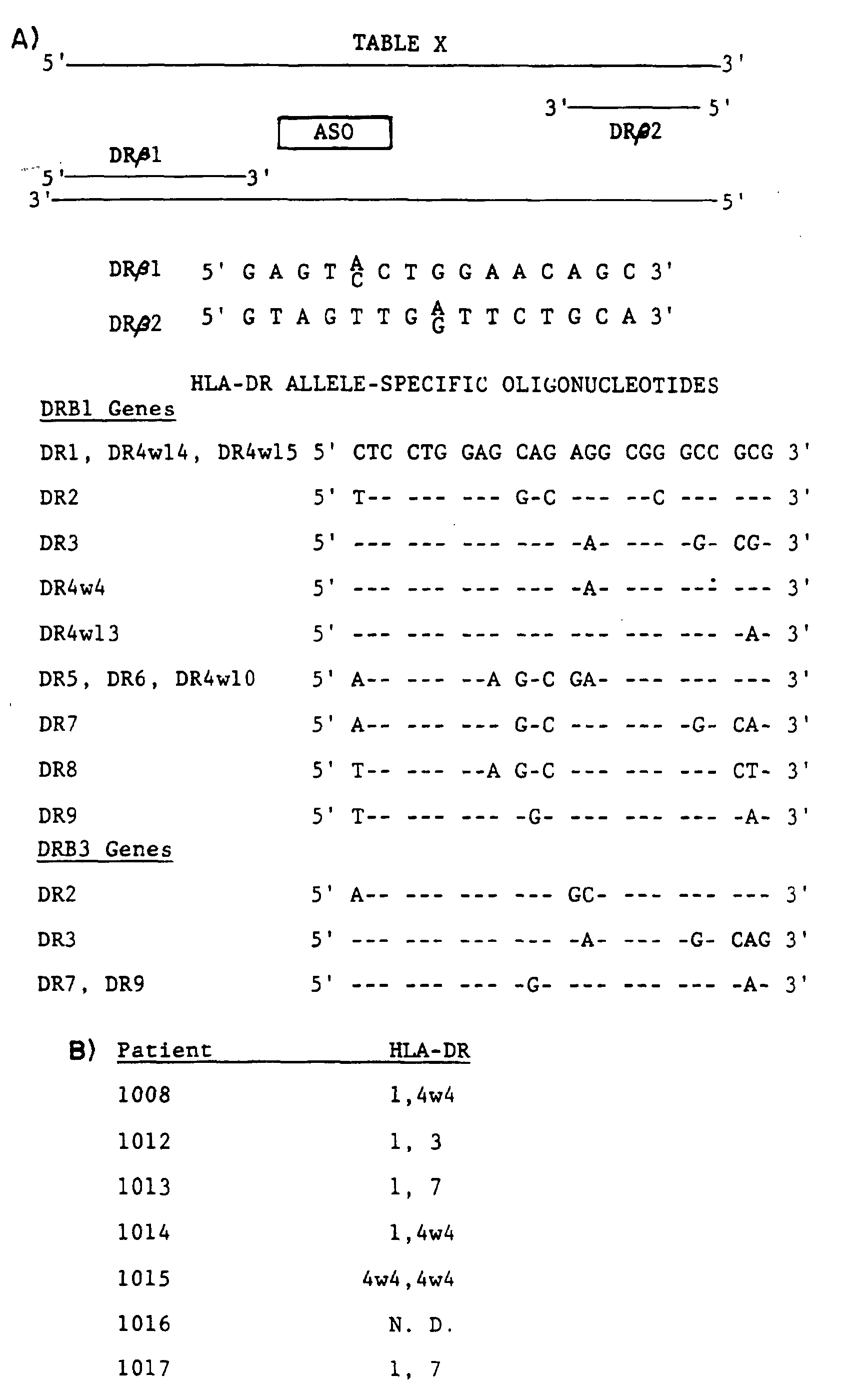

T cell receptors containing Vβ17 or fragments thereof which are immunogenic or can be made immunogenic can be used to immunize human subjects by methods demonstrated by Example VII. Such immunizations can result in an effective immune response.

Although the invention has been described with reference to the presently-preferred embodiment, it should be understood that various modifications can be made without departing from the spirit of the invention. Accordingly, the invention is limited only by the following claims.

## Claims (Claims for the following Contracting State(s): AT, BE, CH, DE, DK, FR, GB, IT, LI, LU, NL, SE)

1. Use of an immunogenically effective amount of a T cell receptor or fragment thereof corresponding to a T cell receptor present on the surface of T cells mediating a pathology or an unregulated clonal replication, wherein the fragment comprises a V region sequence, or a V(D)J junctional or a J junctional region sequence or mixtures thereof and wherein entire T cells and membrane fractions from entire T cells are excluded, for the preparation of a vaccine for preventing or treating said T cell mediated pathology or an unregulated T cell clonal replication in a mammal.

2. The use of claim 1, wherein said variable region sequence is the β-chain variable region.

3. The use of an immunogenically effective amount of a β-chain variable region which comprises substantially the amino acid sequence MSNQVLCCVVLCFLGANTVDGGITQSPKYLFRKEGQNVTLS-CEQNLNHDAMYWYRQDPGQGLRLIYYSQIVNDFQKGDIAEGYSVSREK-KESFPLTVTSAQKNPTAFYLCASS for preparing a vaccine for preventing or treating rheumatoid arthritis.

4. The use of claim 3, wherein the β-chain variable region is the sequence SQIVNDFQK.

5. The use of claims 1 to 4, wherein further an adjuvant is used.

6. The use of claims 1 to 5, wherein more than one type of T cell receptor or fragment thereof is used.

7. The use of claims 1 to 5, wherein more than one fragment corresponding to different sequences of the same T cell receptor is used.

8. The use of claims 1 to 7, wherein said fragment is conjugated to a carrier.

9. A method of selecting a vaccine for use in treating a T cell mediated pathology comprising the steps of:
a. obtaining T cell clones mediating said condition;
b. determining the amino acid sequence of T cell receptors from T cell clones associated with said condition;
c. selecting segments of those T cell receptors comprising a V region sequence, a V(D)J junctional or a J junctional region sequence or mixtures thereof, which are characteristic of said associated T cell receptors but not of non-associated T cell receptors, and wherein entire T cells and membrane fractions from entire T cells are excluded;
d. selecting amino acid sequences of said selected sequences which are capable of eliciting an immunogenic response to said T cell receptor, and thereby selecting the vaccine.

10. A method of diagnosing or predicting susceptibility to rheumatoid arthritis in an individual comprising detecting T cells having the β-chain variable region MSNQVLCCVVLCFLGANTVDGG-ITQSPKYLFRKEGQNVTLSCEQNLNHDAMYWYRQDPGQGLRLIYYSQIVNDFQKGDIAEGYSV-SREKKESFPLTVT-SAQKNPTAFYLCASS or a fragment thereof in a sample from the individual, the presence of abnormal expression of such region containing T cells indicating rheumatoid arthritis or susceptibility to rheumatoid arthritis.

11. The method of claim 10, comprising detecting a portion of said β-chain variable region which substantially does not occur on non-rheumatoid arthritis associated T-cell receptors.

12. The method of claim 10 or 11, wherein said sample is from synovial tissue.

13. The method of claims 10 to 12, wherein said β-chain variable region is detected by contacting said region with a detectable ligand.

14. The method of claims 10 to 13, wherein the presence of said β-chain variable region is detected by a nucleotide probe which is complementary to the nucleotide sequence encoding said region.

15. Use of anti-idiotypic antibodies which are internal images of a T cell receptor mediating a pathology or unregulated T cell clonal replication in a mammal or of a fragment thereof, wherein the fragment comprises a V region sequence, a V(D)J junctional or a J junctional region sequence or mixtures thereof and wherein entire T cells and membrane fractions from entire T cells are excluded, for the preparation of a vaccine for preventing or treating said pathology.

16. The use of claims 15, wherein further an adjuvant is included in the vaccine.

17. The use of claims 15 or 16, wherein anti-idiotype antibodies which are internal images of more than one T cell receptor or fragment thereof are used.

## Claims (Claims for the following Contracting State(s): ES)

1. A process for manufacturing a vaccine for preventing or treating a T cell mediated pathology or an unregulated T cell clonal replication in a mammal, wherein an immunogenically effective amount of a T cell receptor or fragment thereof corresponding to a T cell receptor present on the surface of T cells mediating said pathology, wherein the fragment comprises a V-region sequence, or a V(D)J junctional or a J junctional region sequence or mixtures thereof and wherein entire T cells and membrane fractions from entire T cells are exluded, is selected and formulated in a pharmaceutically acceptable medium to provide a vaccine.

2. The process of claim 1, wherein said variable region sequence is the β-chain variable region.

3. The process of claims 1 or 2, wherein said T cell mediated pathology is rheumatoid arthritis and wherein said β-chain variable region comprises substantially the amino acid sequence MSNQVLCCVVLCFLGANTVDGGITQSPKYLFRKEGQNVTLSCEQNLNHDAMYWYR-QDPGQGLRLIYYSQIVNDFQKGDIAEGYSVSREKKESFPLTVTSAQKNPTAFYLCASS and fragments thereof effective in a vaccine for preventing or treating rheumatoid arthritis.

4. The process of claim 2 or 3, wherein said β-chain variable region is the sequence SQIVNDFQK.

5. The process of claims 1 to 4, further comprising an adjuvant.

6. The process of claims 1 to 5, wherein said vaccine comprises more than one type of T cell receptor or fragment thereof.

7. The process of claims 1 to 5, wherein said vaccine comprises more than one fragment corresponding to different sequences of the same T cell receptor.

8. The process of claims 1 to 7, wherein said fragment is conjugated to a carrier.

9. A method of selecting a vaccine for use in treating a T cell mediated pathology comprising the steps of:
a. obtaining T cell clones mediating said condition;
b. determining the amino acid sequence of T cell receptors from T cell clones associated with said condition;
c. selecting segments of those T cell receptors comprising a V region sequence, a V(D)J junctional or a J junctional region sequence or mixtures thereof, which are characteristic of said associated T cell receptors but not of non-associated T cell receptors, and wherein entire T cells and membrane fractions from entire T cells are exluded; and
d. selecting amino acid sequences of said selected sequences which are capable of eliciting an immunogenic response to said T cell receptor, and thereby selecting the vaccine.

10. A method of diagnosing or predicting susceptibility to rheumatoid arthritis in an individual comprising detecting T cells having the β-chain variable region MSNQVLCCVVLCFLGANTVDGG-ITQSPKYLFRKEGQNVTLSCEQNLNHDAMYWYRQDPGQGLRLIYYSQIVNDFQKGDIAEGYSV-SREKKESFPLTVT-SAQKNPTAFYLCASS or a fragment thereof in a sample from the individual, the presence of abnormal expression of such region containing T cells indicating rheumatoid arthritis or susceptibility to rheumatoid arthritis.

11. The method of claim 10, comprising detecting a portion of said β-chain variable region which substantially does not occur on non-rheumatoid arthritis associated T cell receptors.

12. The method of claims 10 or 11, wherein said sample is from synovial tissue.

13. The method of claims 10 to 12, wherein said β-chain variable region is detected by contacting said region with a detectable ligand.

14. The method of claims 10 to 13, wherein the presence of said β-chain variable region is detected by a nucleotide probe which is complementary to the nucleotide sequence encoding said region.

15. A process for manufacturing a vaccine for preventing or treating a T cell mediated pathology or unregulated T cell clonal replication in a mammal wherein anti-idiotypic antibodies which are internal images of a T cell receptor or a fragment thereof corresponding to a cell receptor present on the surface of a T cell mediating said pathology, wherein the fragment comprises a V-region sequence, a V (D) J junctional or a J junctional region sequence or mixtures thereof and wherein entire T cells and membrane fractions from entire T cells are exluded, are selected and formulated in a pharmaceutically acceptable medium to provide a vaccine.

16. The process of claim 15, further comprising an adjuvant.

17. The process of claim 15, wherein said vaccine comprises anti-idiotype antibodies which are internal images of more than one T cell receptor or fragment thereof.

## Patentansprüche (Patentansprüche für folgende(n) Vertragsstaat(en): AT, BE, CH, DE, DK, FR, GB, IT, LI, LU, NL, SE)

1. Verwendung einer immunogen wirksamen Menge eines T-Zell-Rezeptors, der einem T-Zell-Rezeptor entspricht, der auf der Oberfläche von T-Zellen vorhanden ist, die eine Pathologie oder eine unregulierte klonale Replikation vermitteln, oder eines Fragments davon, wobei das Fragment eine Sequenz einer V-Region, oder einer V(D) J-verknüpfender oder einer J-verknüpfender Region oder Mischungen davon umfaßt und wobei vollständige T-Zellen oder Membranfraktionen von vollständigen T-Zellen ausgeschlossen sind, zur Herstellung eines Impfstoffs zur Prävention oder Behandlung der genannten T-Zell-vermittelten Pathologie oder einer unregulierten klonalen T-Zell-Replikation in einem Säuger.

2. Verwendung nach Anspruch 1, bei der die genannte Sequenz der variablen Region die variable β-Kettenregion ist.

3. Verwendung einer immunogen effektiven Menge der variablen β-Kettenregion, die im wesentlichen die Aminosäuresequenz MSNQVLCCVVLCFLGANTVDGGITQSPKYLFRKEGQNVTLSCEQNLNHDAMYWYRQDP-GQGLRLIYYSQIVNDFQKGDIAEGYSVSREKKESFPLTVTSAQKNPTAFYLCASS umfasst, zur Herstellung eines Impfstoffs zur Prävention oder Behandlung rheumatoider Arthritis.

4. Verwendung nach Anspruch 3, bei der die variable β-Kettenregion die Sequenz SQIVNDFQK ist.

5. Verwendung nach den Ansprüchen 1 bis 4, bei der man ferner ein Adjuvans verwendet.

6. Verwendung nach den Ansprüchen 1 bis 5, bei der man mehr als einen Typ von T-Zell-Rezeptoren oder Fragmenten davon verwendet.

7. Verwendung nach den Ansprüchen 1 bis 5, bei der man mehr als ein Fragment, das unterschiedlichen Sequenzen des selben T-Zell-Rezeptors entspricht, verwendet.

8. Verwendung nach den Ansprüchen 1 bis 7, bei der das genannte Fragment mit einem Träger konjugiert ist.

9. Verfahren zur Auswahl eines Impfstoffs zur Verwendung bei der Behandlung einer T-Zell-vermittelten Pathologie, das die Schritte umfasst:
a. Erhalten von T-Zell-Klonen, die den genannten Zustand vermitteln;
b. Bestimmung der Aminosäuresequenz von T-Zell-Rezeptoren aus T-Zell-Klonen, die mit dem genannten Zustand assoziiert sind;
c. Auswahl von Segmenten derjenigen T-Zell-Rezeptoren, die einer Sequenz einer V-Region, einer V(D)Jverknüpfenden oder einer J-verknüpfenden Region oder Mischungen davon umfassen, die für die genannten assoziierten T-Zell-Rezeptoren, nicht aber für nichtassoziierte T-Zell-Rezeptoren charakteristisch sind, und wobei vollständige T-Zellen und Membranfraktionen von vollständigen T-Zellen ausgeschlossen sind;
d. Auswahl von Aminosäuresequenzen der genannten ausgewählten Sequenzen, die in der Lage sind, eine immunogene Antwort auf den genannten T-Zell-Rezeptor auszulösen, und dadurch Auswahl des Impfstoffs.

10. Verfahren zur Diagnose oder Vorhersage einer Empfänglichkeit für rheumatoide Arthritis in einem Individuum, das das Nachweisen von T-Zellen, die die variable β-Kettenregion MSNQVLCCVVLCFLGANTVDGGITQSPKYLFRKEGQNVTLSCEQNLNHDAMYWYRQDP-GQGLRLIYYSQIVNDFQKGDIAEGYSVSREKKESFPLTVT-SAQKNPTAFYLCASS oder ein Fragment davon haben, in einer Probe aus dem Individuum umfasst, wobei das Vorhandensein einer abnormen Expression von solchen, die Region enthaltenen T-Zellen eine rheumatoide Arthritis oder die Empfänglichkeit für rheumatoide Arthritis anzeigt.

11. Verfahren nach Anspruch 10, das das Nachweisen eines Teils der genannten variablen β-Kettenregion, die auf nicht mit rheumatoider Arthritis assoziierten T-Zell-Rezeptoren im wesentlichen nicht zu finden ist, umfaßt.

12. Verfahren nach den Ansprüchen 10 oder 11, bei dem die genannte Probe aus synovialem Gewebe stammt.

13. Verfahren nach den Ansprüchen 10 bis 12, bei dem die genannte variable β-Kettenregion durch Kontaktieren der genannten Region mit einem detektierbaren Liganden nachgewiesen wird.

14. Verfahren nach den Ansprüchen 10 bis 13, bei dem man das Vorhandensein der genannten variablen β-Kettenregion durch eine Nukleotidsonde nachweist, die zur Nukleotidsequenz, die für die genannte Region kodiert, komplementär ist.

15. Verwendung von anti-idiotypischen Antikörpern, die innere Abbilder eines T-Zell-Rezeptors, der eine Pathologie oder eine unregulierte klonale T-Zell-Replikation in einem Säuger vermittelt, oder eines Fragments davon sind, wobei das Fragment eine Sequenz einer V-Region, einer V(D)Jverknüpfenden oder einer J-verknüpfenden Region oder Mischungen davon umfasst, und wobei vollständige T-Zellen und Membranfraktionen von vollständigen T-Zellen ausgeschlossen sind, zur Herstellung eines Impfstoffs zur Prävention oder Behandlung der genannten Pathologie.

16. Verwendung nach Anspruch 15, bei der in den Impfstoff ferner ein Adjuvans eingeschlossen ist.

17. Verwendung nach den Ansprüchen 15 oder 16, bei der man anti-idiotypische Antikörper verwendet, die innere Abbilder von mehr als einem T-Zell-Rezeptor oder Fragment davon sind.

## Patentansprüche (Patentansprüche für folgende(n) Vertragsstaat(en): ES)

1. Verfahren zur Herstellung eines Impfstoffs zur Prävention oder Behandlung einer T-Zell-vermittelten Pathologie oder einer unregulierten klonalen T-Zell-Replikation in einem Säuger, bei dem man eine immunogen effektive Menge eines T-Zell-Rezeptors, der einem T-Zell-Rezeptor entspricht, der auf der Oberfläche von T-Zellen, die die genannte Pathologie vermitteln, vorhanden ist, oder Fragments davon, wobei das Fragment eine Sequenz einer V-Region oder einer V(D)Jverknüpfenden oder einer J-verknüpfenden Region oder Mischungen davon umfaßt und wobei vollständige T-Zellen und Membranfraktionen von vollständigen T-Zellen ausgeschlossen sind, auswählt und in einem pharmazeutisch akzeptablen Medium formuliert, um einen Impfstoff bereitzustellen.

2. Verfahren nach Anspruch 1, bei dem die genannte variable Region die variable β-Kettenregion ist.

3. Verfahren nach den Anspruchen 1 oder 2, bei dem die genannte T-Zell-vermittelte Pathologie rheumatoide Arthritis ist und bei dem die genannte variable β-Kettenregion im wesentlichen die Aminosäuresequenz MSNQVLCCVVLCFLGANTVDGGITQSPK-YLFRKEGQNVTLSCEQNLNHDAMYWYRQDPGQGLRLIYYSQIVNDFQKGDIAEGYSVSR EKKESFPLTVTSAQKNPTAFYLCASS und Fragmente davon umfaßt, die wirksam in einem Impfstoff zur Verhinderung oder Behandlung von rheumatoider Arthritis sind.

4. Verfahren nach Anspruch 2 oder 3, bei dem die genannte variable β-Kettenregion die Sequenz SQIVNDFQK ist.

5. Verfahren nach den Ansprüchen 1 bis 4, das ferner ein Adjuvans umfasst.

6. Verfahren näch den Ansprüchen 1 bis 5, bei dem der genannte Impfstoff mehr als einen Typ von T-Zell-Rezeptoren oder Fragmenten davon umfasst.

7. Verfahren nach den Ansprüchen 1 bis 5, bei dem der genannte Impfstoff mehr als ein Fragment, das verschiedenen Sequenzen des selben T-Zell-Rezeptors entspricht, umfasst.

8. Verfahren nach den Ansprüchen 1 bis 7, bei dem das genannte Fragment mit einem Träger konjugiert ist.

9. Verfahren zur Auswahl eines Impfstoffs zur Verwendung bei der Behandlung einer T-Zell-vermittelten Pathologie, das die Schritte umfaßt:
a. Erhalten von T-Zell-Klonen, die den genannten Zustand vermitteln;
b. Bestimmung der Aminosäuresequenz von T-Zell-Rezeptoren aus T-Zell-Klonen, die mit dem genannten Zustand assoziiert sind;
c. Auswahl von Segmenten derjenigen T-Zell-Rezeptoren, die einer Sequenz einer V-Region, einer V(D)Jverknüpfenden oder einer J-verknüpfenden Region oder Mischungen davon umfassen, die für die genannten assoziierten T-Zell-Rezeptoren, nicht aber für nichtassoziierte T-Zell-Rezeptoren charakteristisch sind, und wobei vollständige T-Zellen und Membranfraktionen von vollständigen T-Zellen ausgeschlossen sind;
d) Auswahl von Aminosäuresequenzen der genannten ausgewählten Sequenzen, die in der Lage sind, eine immunogene Antwort auf den genannten T-Zell-Rezeptor auszulösen, und dadurch Auswahl des Impfstoffs.

10. Verfahren zur Diagnose oder Vorhersage einer Empfänglichkeit für rheumatoide Arthritis in einem Individuum, das das Nachweisen von T-Zellen, die die variable β-Kettenregion MSNQVLCCVVLCFLGANTVDGGITQSPKYLFRKEGQNVTLSCEQNLNHDAMYWYRQDP-GQGLRLIYYSQIVNDFQKGDIAEGYSVSREKKESFPLTVT-SAQKNPTAFYLCASS oder ein Fragment davon haben, in einer Probe aus dem Individuum umfaßt, wobei das Vorhandensein einer abnormen Expression von solchen, die Region enthaltenen T-Zellen eine rheumatoide Arthritis oder die Empfänglichkeit für rheumatoide Arthritis anzeigt.

11. Verfahren nach Anspruch 10, das das Nachweisen eines Teils der genannten variablen β-Kettenregion, die auf nicht mit rheumatoider Arthritis assoziierten T-Zell-Rezeptoren im wesentlichen nicht zu finden ist, umfaßt.

12. Verfahren nach den Ansprüchen 10 oder 11, bei dem die genannte Probe aus synovialem Gewebe stammt.

13. Verfahren nach den Ansprüchen 10 bis 12, bei dem die genannte variable β-Kettenregion durch Kontaktieren der genannten Region mit einem detektierbaren Liganden nachgewiesen wird.

14. Verfahren nach den Ansprüchen 10 bis 13, bei dem man das Vorhandensein der genannten variablen β-Kettenregion durch eine Nukleotidsonde nachweist, die zur Nukleotidsequenz, die für die genannte Region kodiert, komplementär ist.

15. Verfahren zur Herstellung eines Impfstoffs zur Prävention oder Behandlung einer T-Zell-vermittelten Pathologie oder unregulierten klonalen T-Zell-Replikation in einem Säuger, bei dem man anti-idiotypische Antikörper, die innere Abbilder eines T-Zell-Rezeptors, der einem Zell-Rezeptor entspricht, der auf der Oberfläche einer T-Zelle- vorhanden ist, die die genannte Pathologie vermittelt, oder Fragments davon sind, wobei das Fragment eine Sequenz einer V-Region, einer V(D)J-verknüpfenden oder einer J-verknüpfenden Region oder Mischungen davon umfaßt und bei dem vollständige T-Zellen und Membranfraktionen von vollständigen T-Zellen ausgeschlossen sind, auswählt und in einem pharmazeutisch akzeptablen Medium formuliert, um einen Impfstoff bereitzustellen.

16. Verfahren nach Anspruch 15, das ferner ein Adjuvans umfaßt.

17. Verfahren nach Anspruch 15, bei dem der genannte Impfstoff anti-idiotypische Antikörper umfasst, die innere Abbilder von mehr als einem T-Zell-Rezeptor oder Fragment davon sind.

## Revendications (Revendications pour l'(les) Etat(s) contractant(s) suivant(s): AT, BE, CH, DE, DK, FR, GB, IT, LI, LU, NL, SE)

1. Utilisation d'une quantité immunogéniquement efficace d'un récepteur de cellules T ou son fragment correspondant à un récepteur de cellules T présent à la surface de cellules T médiatrices d'une pathologie ou d'une réplication clonale non régulée, où le fragment comprend une séquence de région V ou une séquence d'une région de jonction V(D)J ou une séquence d'une région de jonction J ou des mélanges de celles-ci et où des cellules T entières et des fractions de membrane de cellules T sont exclues, pour la préparation d'un vaccin pour la prévention ou le traitement de ladite pathologie provoquée par les cellules T ou une réplication clonale des cellules T non régulée chez un mammifère.

2. Utilisation de la revendication 1, où ladite séquence de région variable est la région variable de la chaîne β.

3. Utilisation d'une quantité immunogéniquement efficace d'une région variable de chaîne β qui comprend sensiblement la séquence d'acides aminés MSNQVLCCVVLCFLGANTVDGGITQSPKYLFRKEGQNVTLSCEQNLNHDAMYWYRQDPGQG LRLIYYSQIVNDFQKGDIAEGYSVSREKKESFPLTVTSAQKNPTAFYLCASS pour la préparation d'un vaccin pour la prévention ou le traitement de l'arthrite rhumatoïde.

4. Utilisation de la revendication 3, où la région variable de chaîne β est la séquence SQIVNDFQK.

5. Utilisation des revendications 1 à 4, où de plus un adjuvant est utilisé.

6. Utilisation des revendications 1 à 5, où plus d'un type de récepteur de cellules T ou fragment de celui-ci est utilisé.

7. Utilisation des revendications 1 à 5, où plus d'un fragment correspondant à différentes séquences du même récepteur de cellules T est utilisé.

8. Utilisation des revendications 1 à 7, où ledit fragment est conjugué à un support.

9. Méthode de sélection d'un vaccin à utiliser dans le traitement d'une pathologie provoquée par les cellules T comprenant les étapes de :
a. obtenir des clones de cellules T provoquant ladite condition ;
b. déterminer la séquence d'acides aminés des récepteurs de cellules T de clones de cellules T associés à ladite condition ;
c. sélectionner des segments des récepteurs de cellules T comprenant une séquence de région V, une séquence de région de jonction V(D)J ou de jonction J ou des mélanges de celles-ci, qui sont caractéristiques desdits récepteurs de cellules T associés mais non pas des récepteurs de cellules T non associés, et où des cellules T entières et des fractions de membrane de cellules T entières sont exclues ; et
d. sélectionner des séquences d'acides aminés desdites séquences sélectionnées qui sont capables d'éliciter une réponse immunogène audit récepteur de cellules T et ainsi sélectionner le vaccin.

10. Méthode de diagnostic ou de prédiction d'une sensibilité à l'arthrite rhumatoïde chez un individu comprenant la détection de cellules T ayant la région variable de chaîne β MSNQVLCCVVLCFLGANTVDGGITQSPKYLFRK EGQNVTLSCEQNLNHDAMYWYRQDPGQGLRLIYYSQIVNDFQKGDIAEGYSVSREKKESFP LTVT-SAQKNPTAFYLCASS ou un fragment de celle-ci dans un échantillon de l'individu, la présence d'une expression anormale de cellules T contenant une telle région indiquant l'arthrite rhumatoïde ou la sensibilité à l'arthrite rhumatoïde.

11. Méthode de la revendication 10, comprenant la détection d'une portion de ladite région variable de chaîne β qui ne se présente sensiblement pas sur des récepteurs de cellules T associés à une arthrite non rhumatoïde.

12. Méthode de la revendication 10 ou 11, où ledit échantillon est d'un tissu synovial.

13. Méthode des revendications 10 à 12, où ladite région variable de chaîne β est détectée par mise en contact de ladite région avec un ligand détectable.

14. Méthode des revendications 10 à 13, où la présence de ladite région variable de chaîne β est détectée par une sonde nucléotidique qui est complémentaire de la séquence de nucléotides codant pour ladite région.

15. Utilisation d'anticorps anti-idiotypiques qui sont des images internes d'un récepteur de cellules T provoquant une pathologie ou une réplication clonale non régulée des cellules T chez un mammifère ou d'un fragment de ceux-ci où le fragment comprend une séquence de région V, une séquence de région de jonction V(D)J ou de jonction J ou des mélange de celles-ci et où des cellules T entières et des fractions de membrane de cellules T entières sont exclues, pour la préparation d'un vaccin pour la prévention ou le traitement de ladite pathologie.

16. Utilisation de la revendication 15, où de plus un adjuvant est inclus dans le vaccin.

17. Utilisation des revendications 15 ou 16, où des anticorps anti-idiotypiques qui sont des images internes de plus d'un récepteur de cellules T ou fragment de celui-ci sont utilisés.

## Revendications (Revendications pour l'(les) Etat(s) contractant(s) suivant(s): ES)

1. Procédé pour la fabrication d'un vaccin pour la prévention ou le traitement d'une pathologie provoquée par des cellules T ou d'une réplication clonale non régulée de cellules T chez un mammifère, où une quantité immunogéniquement efficace d'un récepteur de cellules T ou fragment de celui-ci correspondant à un récepteur de cellules T présent à la surface de cellules T médiatrices d'une pathologie ou d'une réplication clonale non régulée, où le fragment comprend une séquence de région V ou une séquence d'une région de jonction V(D)J ou une séquence d'une région de jonction J ou des mélanges de celles-ci et où des cellules T entières et des fractions de membrane de cellules T sont exclues, pour la préparation d'un vaccin pour la prévention ou le traitement de ladite pathologie provoquée par les cellules T ou une réplication clonale des cellules T non régulée chez un mammifère.

2. Procédé de la revendication 1 où ladite séquence de la région variable est la région variable de la chaîne β.

3. Procédé des revendications 1 ou 2, où ladite pathologie provoquée par les cellules T est l'arthrite rhumatoïde et où ladite région variable de chaîne β comprend sensiblement la séquence d'acides aminés MSNQVLCCVVLCFLGANTVDGGITQSPKYLFRKEGQNVTLSCEQNLNHDAMYWYRQDPGQG LRLIYYSQIVNDFQKGDIAEGYSVSREKKESFPLTVTSAQKNPTAFYLCASS et des fragments de celle-ci efficaces dans un vaccin pour prevenir ou traiter l'arthrite rhumatoïde.

4. Procédé de la revendication 2 ou 3, où ladite région variable de chaîne β est la séquence SQIVNDFQK.

5. Procédé des revendications 1 à 4, comprenant de plus un adjuvant.

6. Procédé des revendications 1 à 5, où ledit vaccin comprend plus d'un type de récepteur de cellules T ou fragment de celui-ci.

7. Procédé des revendications 1 à 5, où ledit vaccin comprend plus d'un fragment correspondant à différentes séquences du même récepteur de cellules T.

8. Procédé des revendications 1 à 7, où ledit fragment est conjugué à un support.

9. Méthode de sélection d'un vaccin à utiliser dans le traitement d'une pathologie provoquée par les cellules T comprenant les étapes de :
a. obtenir des clones de cellules T provoquant ladite condition ;
b. déterminer la séquence d'acides aminés des récepteurs de cellules T de clones de cellules T associés à ladite condition ;
c. sélectionner des segments des récepteurs de cellules T comprenant une séquence de région V, une séquence de région de jonction V(D)J ou de jonction J ou des mélanges de celles-ci, qui sont caractéristiques desdits récepteurs de cellules T associés mais non pas des récepteurs de cellules T non associés, et où des cellules T entières et des fractions de membrane de cellules T entières sont exclues ;
d. sélectionner des séquences d'acides aminés desdites séquences sélectionnées qui sont capables d'éliciter une réponse immunogène audit récepteur de cellules T et ainsi sélectionner le vaccin.

10. Méthode de diagnostic ou de prédiction d'une sensibilité à l'arthrite rhumatoïde chez un individu comprenant la détection de cellules T ayant la région variable de chaîne β MSNQVLCCVVLCFLGANTVDGGITQSPKYLFRK EGQNVTLSCEQNLNHDAMYWYRQDPGQGLRLIYYSQIVNDFQKGDIAEGYSVSREKKESFP LTVT-SAQKNPTAFYLCASS ou un fragment de celle-ci dans un échantillon de l'individu, la présence d'une expression anormale de cellules T contenant une telle région indiquant l'arthrite rhumatoïde ou la sensibilité à l'arthrite rhumatoïde.

11. Méthode de la revendication 10, comprenant la détection d'une portion de ladite région variable de chaîne β qui ne se présente sensiblement pas sur des récepteurs de cellules T associés à une arthrite non rhumatoïde.

12. Méthode de la revendication 10 ou 11, où ledit échantillon est d'un tissu synovial.

13. Méthode des revendications 10 à 12, où ladite région variable de chaîne β est détectée par mise en contact de ladite région avec un ligand détectable.

14. Méthode des revendications 10 à 13, où la présence de ladite région variable de chaîne β est détectée par une sonde nucléotidique qui est complémentaire de la séquence de nucléotides codant pour ladite région.

15. Procédé pour la fabrication d'un vaccin pour la prévention ou le traitement d'une pathologie provoquée par les cellules T ou d'une réplication clonale non régulée des cellules T chez un mammifère où des anticorps anti-idiotypiques qui sont des images internes d'un récepteur de cellules T ou d'un fragment de celui-ci correspondant à un récepteur de cellules présent à la surface d'une cellule T provoquant ladite pathologie, où le fragment comprend une séquence de région V, une séquence de région de jonction V(D)J ou de jonction J ou leurs mélanges et où des cellules T entières et des fractions de membrane de cellules T entières sont exclues, sont sélectionnés et formulés dans un milieu pharmaceutiquement acceptable pour produire un vaccin.

16. Procédé de la revendication 15, comprenant de plus un adjuvant.

17. Procédé de la revendication 15, où ledit vaccin comprend des anticorps anti-idiotypiques qui sont des images internes de plus d'un récepteur de cellules T ou fragment de celui-ci.
